# EUROPÄISCHE PATENTSCHRIFT

(11) **EP 2 218 469 B1**
(45) Veröffentlichungstag und Bekanntmachung des Hinweises auf die Patenterteilung: **31.10.2012**
(21) Anmeldenummer: 09075071.2
(22) Anmeldetag: 12.02.2009
(51) Int. Cl.: A61M 1/10, A61M 1/12

(54) **Gehäuse für ein Funktionselement**
Casing for a functional element
Boîtier pour un élément fonctionnel

(43) Veröffentlichungstag der Anmeldung: 18.08.2010
(73) Patentinhaber: ECP Entwicklungsgesellschaft mbH, 12247 Berlin (DE)
(72) Erfinder: Scheckel, Mario, 13187 Berlin (DE)
(74) Vertreter: Pfenning, Meinig & Partner GbR

(56) Entgegenhaltungen:
- WO-A-94/05347
- DE-A1-102004 054 714
- US-A1- 2008 132 748

## Beschreibung

Die Erfindung liegt auf dem Gebiet des Maschinenbaus und der Feinmechanik und ist insbesondere im medizinischen Bereich mit Vorteil einsetzbar.

Besonders auf dem Gebiet der Medizin werden an Gehäuse für Funktionselemente, die beispielsweise im invasiven bzw. mikroinvasiven Betrieb verwendet werden, hohe Anforderungen gestellt. Solche Gehäuse müssen oft ebenso wie die entsprechenden Funktionselemente sehr klein sein, um möglichst bei einem Einführen in den Körper eines Patienten keinen Schaden anzurichten bzw. kleine Eintrittswunden zu erfordern. So werden mikroinvasive Eingriffe möglich, um beispielsweise Körperfunktionen wie den Blutkreislauf, d.h. die Pumpleistung des Herzens zu unterstützen oder ähnliches.

Insbesondere für Mikropumpen, die im Patientenkörper betrieben werden können, sind Pumpengehäuse bekannt, die zusammen mit der jeweiligen Pumpe über ein Blutgefäß in den Körper eingeführt werden können. Zu diesem Zweck sind oft sowohl die Förderschaufeln der Pumpe als auch das Pumpengehäuse verformbar zwischen einem komprimierten Zustand zur Bewegung durch ein Körpergefäß und einem erweiterten Zustand, in dem die Pumpe planmäßig betrieben wird.

Aus der Literatur sind verschiedene Ansätze dafür bekannt, wie derartige Bauteile gesteuert im Körper komprimiert oder expandiert werden können. Beispielsweise ist bekannt, so genannte Formgedächtnismaterialien zu verwenden, die beispielsweise durch Temperaturänderungen in verschiedene Formen gebracht werden können. Im Zusammenhang mit der Verwendung von Formgedächtnismaterialien ist jedoch zu beachten, dass diese teilweise schwierig zu beherrschende, oft nichtlineare mechanische Eigenschaften aufweisen und zudem kostenintensiv sind.

Aus dem Patentdokument DE 10 2004 054 714 A1 ist eine Lösung bekannt, bei der sowohl das Laufrad einer Mikropumpe als auch deren Gehäuse durch eine relative axiale Verschiebung der Pumpenantriebswelle gegenüber einem Katheter expandiert wird. Hierdurch wird das Gehäuse zwischen dem komprimierten und dem expandierten Zustand gefaltet. Dieses Dokument zeigt keine Membran, an welcher Formkörper befestigt sind. Stattdessen zeigt die DE 10 2004 054 714 A1 (siehe beispielsweise Fig. 6) einen Rotor mit relativ steifen Auslegern, der im Betrieb an einer Gehäusemembran schleift. Die Gehäusemembran wird somit durch den sich aufrichtenden Rotor aufgespreizt.

Die US 2008/132748 A1 zeigt eine entfaltbare Pumpvorrichtung (siehe beispielsweise Fig. 4a für den expandierten und Fig. 4b für den komprimierten Zustand des Pumpenkopfs). Dieses Dokument zeigt allerdings zumindest nicht das Merkmal, dass eine Fluidpumpe in dem Gehäuse einen Überdruck erzeugt, der zum Aufspannen der Membran führt und somit die Gehäuseform beeinflusst.

Aus der WO 00/2003103745 A2 ist ein System bekannt, bei dem das Pumpengehäuse ebenfalls durch eine axiale Relativbewegung zweier Bauteile gegeneinander radial expandiert wird,

Der vorliegenden Erfindung liegt vor dem Hintergrund des Standes der Technik die Aufgabe zugrunde, ein Gehäuse zu schaffen, dass mit möglichst einfachen Mitteln komprimierbar bzw. expandierbar ist, wobei der konstruktive Aufwand minimiert werden soll. Dennoch soll das Gehäuse die für den Betrieb erforderliche Stabilität aufweisen.

Die Aufgabe wird gemäß der Erfindung mit den Merkmalen des Patentanspruchs 1 gelöst.

Die Erfindung bezieht sich auf ein Gehäuse für ein Funktionselement, insbesondere für den Einsatz in körpereigenen Gefäßen im medizinischen Bereich, dessen Gehäusewand eine biegeschlaffe, aufspannbare Membran aufweist mit mehreren an dieser befestigten Formkörpern, die die Membran in deren aufgespanntem Zustand stützen. Alle Bauteile sind dazu vorteilhaft aus biokompatiblen Materialien ausgeführt.

Die entsprechenden Formkörper sind vorteilhaft formstabil und fest und können als Spritzgussteile, insbesondere aus Kunststoff, ausgebildet und im Wesentlichen stab- oder plattenförmig sein.

Weiter kann die Erfindung vorteilhaft dadurch ausgestaltet sein, dass wenigstens einer der Formkörper eine Stützfläche aufweist, die im aufgespannten Zustand an der Membran anliegt.

Es können auch mehrere der Formkörper im aufgespannten Zustand nebeneinander flach an der Membran anliegen, insbesondere an deren Innenseite, um eine Auskleidung der Membran zu bilden, die diese stützt und innerhalb des Gehäuses einen Fluidstrom nicht behindert.

Die Formkörper können vorteilhaft ausschließlich mit der Membran verbunden sein, d.h. keine Verbindung mit einem weiteren Bauteil im Gehäuseinneren haben. Die Positionierung des Gehäuses in Bezug auf ein Funktionselement, insbesondere eine Pumpe, kann dann durch Stützarme realisiert werden, die weiter unten beschrieben sind.

Die Formkörper können mit ihrer gesamten Stützfläche, die an der Membran anliegt, fest verbunden sein oder auch nur mit einem Teil der Stützfläche, insbesondere mit einer Begrenzungskante der Stützfläche verbunden sein.

Die Verbindung der Formkörper mit der Membran kann beispielsweise als Klebeverbindung realisiert sein.

Im aufgespannten Zustand der Membran können die an der Innenseite des Gehäuses/der Membran anliegenden Formkörper die Membran im Wesentlichen abdecken und auch aneinander anliegen.

Dadurch wird die Membran versteift und vor Beschädigungen von der Innenseite her effektiv geschützt.

Die Formkörper können einander teilweise überdecken und gleichsam schuppenartig oder schindelartig übereinander liegen. Damit ist eine besonders gute Stützung und lückenlose Abdeckung der Membran gewährleistet.

Die Formkörper können auch teilweise ineinander greifen, um sich gegenseitig zu stützen und zu positionieren.

Somit können die Formkörper im aufgespannten Zustand des Gehäuses einen eigenstabilen Stützkörper bilden, der die Membran von innen stützt.

Die Erfindung bezieht sich zudem auch auf ein Gerät mit einem Gehäuse nach der oben beschriebenen Art und mit einem Funktionselement, wobei das Gerät eine Fluidpumpe aufweist, die in dem Gehäuse einen Überdruck erzeugt, der zum Aufspannen der Membran führt.

Somit wird die Beweglichkeit der biegeschlaffen Membran und die Beweglichkeit der Formkörper im nicht aufgespannten Zustand der Membran dazu genützt, mittels eines Überdrucks im Gehäuse ohne eine nennenswerte Gegenkraft die Membran zu spannen und diese durch die im gespannten Zustand anliegenden Formkörper zu stabilisieren und zu stützen. Gegebenenfalls muss beim Anlaufen der Pumpe, wenn diese im Gehäuse angeordnet ist, in Kauf genommen werden, dass die Förderschaufeln der Pumpe an Teilen des Gehäuses schleifen, bis durch den sich aufbauenden Überdruck das Gehäuse planmäßig expandiert ist und ein Pumpenspalt zwischen Schaufelspitze und Gehäuse ausgebildet ist.

Wird die Pumpe wieder ausgeschaltet, so kollabiert das Gehäuse und kann gemeinsam mit dem Funktionselement durch das körpereigene Gefäß bzw. ein künstliches Gefäß - z.B. eine Schleuse - zurückgezogen werden.

Im Gegensatz zu anderen Konzepten für komprimierbare und expandierbare Gehäuse wird der Kompressionsbewegung des Gehäuses nach dem Stillsetzen der Pumpe keine nennenswerte elastische Gegenkraft entgegengesetzt, die den Kompressionsvorgang erschweren würde. Besonders vorteilhaft gestaltet sich das beschriebene Verhalten beim Zurückziehen in ein künstliches Gefäß und beim Transport durch dieses Gefäß, da hierbei wenig Kraft für die translatorische Bewegung durch das Gefäß aufgewendet werden muss.

Die Expansion bzw. Kompression des Gehäuses kann, wenn in diesem ein von einer Pumpe verschiedenes Funktionselement angeordnet ist, auch dadurch realisiert werden, dass das Gehäuse mit einer außerhalb angeordneten Fluidpumpe druckbeaufschlagt wird bzw. zum Kollabieren der Druck heruntergefahren wird.

Eine typische Anwendung für die Erfindung ist die Realisierung bei einer Blutpumpe, so dass im Betrieb die Pumpe Blut fördert und damit einen Innendruck aufbaut, der das Gehäuse beispielsweise in einer Herzkammer wunschgemäß expandiert.

Befindet sich in dem Gehäuse eine Fluidpumpe, so sind vorteilhaft eine Ansaugöffnung sowie ein Katheteranschluss vorgesehen. Die Ansaugöffnung kann beispielsweise einen Ansaugkäfig aufweisen, der einerseits koagulierte Blutbestandteile von der Pumpe fernhält und andererseits das körpereigene Gewebe außerhalb des Gehäuses vor einer Verletzung durch die Pumpenförderschaufeln schützt.

Zur Positionierung und Zentrierung des Gehäuses gegenüber einem in diesem befindlichen Funktionselement, insbesondere einem Pumpenrotor, dienen vorteilhaft Stützarme. Diese können sich beispielsweise vom Gehäuse radial bis zu dem Funktionselement oder gegebenenfalls bis zu einer Antriebswelle oder einem auf dieser gelagerten Bauteil hin erstrecken und dort und an mindestens einem Formkörper abgestützt und /oder an der Membran befestigt sein.

In dem Fall, dass das radial innere Ende der Stützarme in axialer Richtung festgelegt ist, ergibt sich im Zuge der Kompressions-/Expansionsbewegung gleichzeitig eine Axialbewegung des Gehäuses, wenn nicht die Stützarme biegbar oder mit wenigstens einem Knickgelenk versehen sind.

Die Stützarme können vorteilhaft im expandierten Zustand des Gehäuses einrasten, wobei die entsprechenden Rastvorrichtungen derart gestaltet sein sollten, dass ein Einknicken der Stützarme erfolgt, sobald der Überdruck im Pumpengehäuse unter einen bestimmten Schwellwert sinkt.

Damit üben die Stützarme im expandierten Zustand des Gehäuses eine zusätzliche Stützwirkung aus.

Auch wenn jeder Stützarm mit mehreren Gelenken versehen ist, können diese in einem entsprechenden Winkel, der dem Soll-Zustand im expandierten Zustand des Gehäuses entspricht, stabil einrasten bis das Gehäuse durch Absinken des Überdrucks kollabiert. Dann können die Stützarme zur Komprimierung des Gehäuses einknicken.

Die Stützarme können im Einlassbereich des Gehäuses, in dem ein Fluid angesaugt wird, auch gleichzeitig einen Ansaugkäfig bilden.

Die Erfindung bezieht sich außer auf ein Gehäuse der beschriebenen Art bzw. ein Gerät mit einem solchen Gehäuse zudem noch auf ein Verfahren zur Herstellung eines Gehäuses, bei dem zunächst auf einer flachen Membran Formkörper befestigt werden und danach die Membran zu einem Schlauch gerollt und zusammengefügt wird.

Auf diese Weise können die Formkörper in einem automatisierten Verfahren leicht auf der Membran, beispielsweise durch Kleben, befestigt werden, ohne dass Platzprobleme diesen Vorgang behindern. Das Gehäuse wird danach aus der flachen Membran als Schlauch gefertigt, der an einem seiner Enden vorteilhaft konisch zu einem Katheteranschluss spitz zulaufen kann. Am entgegen gesetzten Ende des Schlauches können Stützarme zur Bildung eines Ansaugkäfigs vorgesehen sein.

Im Folgenden wird die Erfindung anhand eines Ausführungsbeispiels in einer Zeichnung gezeigt und nachfolgend erläutert.

### Dabei zeigt

- Fig. 1: grundsätzlich den Aufbau eines durch ein Blutgefäß in eine Herzkammer eingeführten Katheters mit einer Herzpumpe,
- Fig. 2: in einer Seitenansicht das Gehäuse im komprimierten Zustand (oben) und im expandier-ten Zustand (unten),
- Fig. 3: eine Seitenansicht eines anderen Gehäuses im komprimierten Zustand (oben) und im expandierten Zustand (unten),
- Fig. 4: das Gehäuse im komprimierten Zustand in einem Querschnitt,
- Fig. 5: das Gehäuse in einem teilweise expandierten Zustand in einem Querschnitt,
- Fig. 6: das Gehäuse im expandierten Zustand im Querschnitt,
- Fig. 7: das Gehäuse in teilweise expandiertem Zustand mit einer eingebauten Pumpe,
- Fig. 8: das Gehäuse im expandierten Zustand mit einer expandierten Pumpe in einem Querschnitt,
- Fig. 9: eine dreidimensionale Ansicht der Membran im flachen Zustand mit aufgeklebten Formkörpern, sowie
- Fig. 10: die Formkörper an der Membran im expandierten Zustand mit einer Darstellung der ineinandergreifenden Enden der Formkörper.

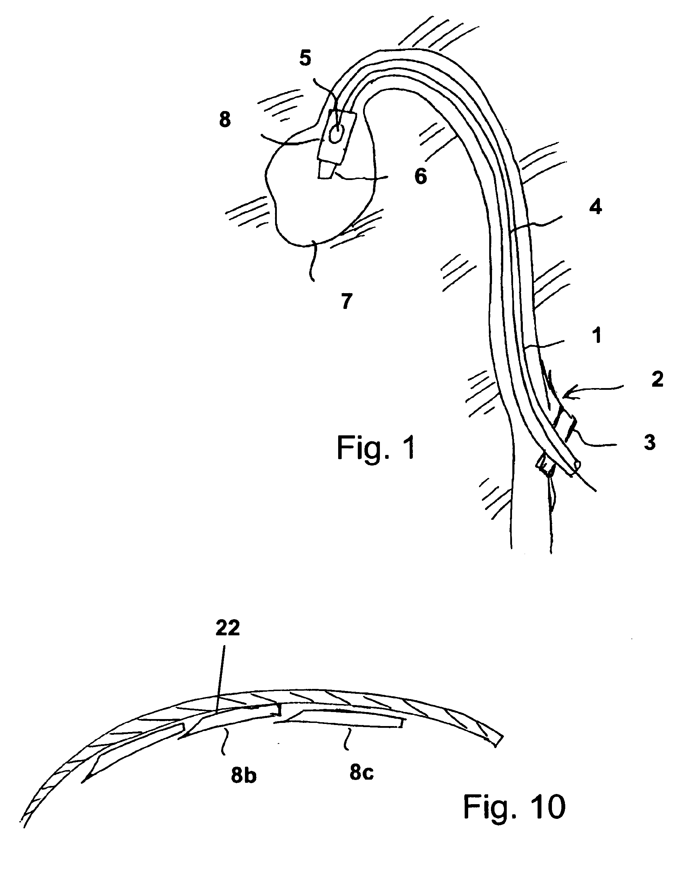

Fig. 1 zeigt schematisch in einem Längsschnitt einen Katheter 1, der in ein Blutgefäß 2 eines menschlichen Körpers mittels einer Schleuse 3 eingeführt und durch dieses Gefäß hindurch bis in die Herzkammer 7 geschleust ist.

Am Ende des Katheters 1 befindet sich ein Funktionselement 5 mit einem Gehäuse 8, wobei das Funktionselement 5 aus einer Blutpumpe mit einem Pumpenrad besteht und das Gehäuse 8 einen Ansaugkäfig 6 an seinem Ende aufweist.

Innerhalb des Katheters 1 ist eine Welle 4 vorgesehen, die bis zu der Pumpe 5 hin verläuft und dort das Pumpenrad mit den Förderschaufeln antreibt.

Das Gehäuse 8 ist in einem wenigstens teilweise expandierten Zustand gezeigt, den es nach dem Einbringen in die Herzkammer und der Betriebsaufnahme der Pumpe einnimmt.

Das Gehäuse und die Pumpe werden im Folgenden anhand der übrigen Figuren detaillierter beschrieben.

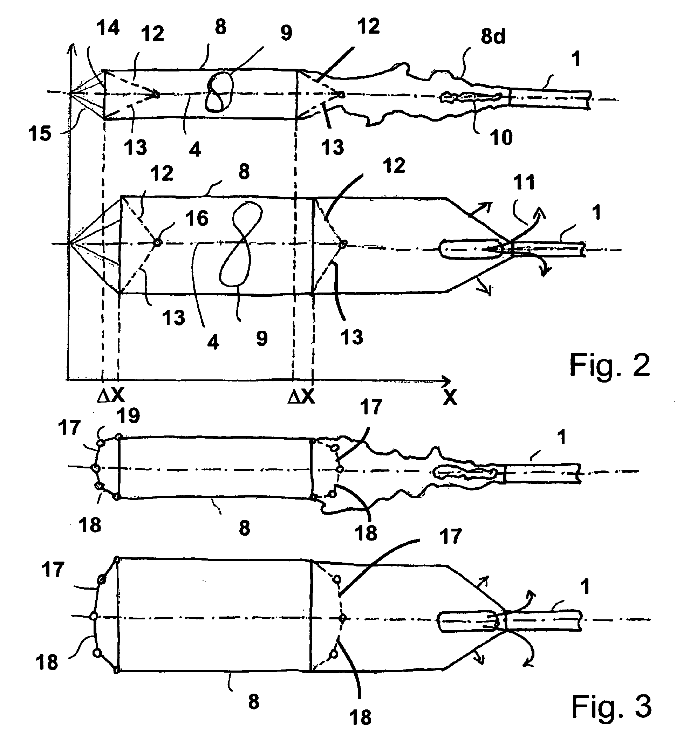

In der Fig. 2 ist oben im komprimierten Zustand eine Seitenansicht des Gehäuses 8 dargestellt, das mit dem Katheter 1 verbunden ist und ein Pumpenlaufrad 9 in ebenfalls komprimiertem Zustand in seinem Inneren aufnimmt. Das Pumpenlaufrad kann beispielsweise eine Nabe und an diese angeklappte Förderschaufeln aufweisen.

Das Gehäuse 8 weist einen konisch spitz zulaufenden Teil 8d auf, der an den Katheter 1 angeschlossen ist und der im Bereich vor dem Katheter mindestens eine Öffnung 10 aufweist, über die die Flüssigkeit ausströmen kann. Dies ist für mehrere Öffnungen durch die Pfeile 11 dargestellt. Somit kann der Transport des Fluids von einem Ort geringeren Druckes - in der Herzkammer - zu einem Ort bei dem der Druck lokal durch den Energieeintrag der Pumpe erhöht wird - oberhalb des Aortensinus (sinus aortae) realisiert werden. Die vor der Öffnung 10 gelagerte Aortenklappe (valva aortae) fungiert als Ventil und hindert das Fluid am Rückströmen in die Herzkammer 7.

Das Gehäuse 8 ist im Wesentlichen zumindest im expandierten Zustand (unten) zylindrisch aufgebaut und besteht in seinem Außenbereich aus einer Membran, die mit Formkörpern an ihrer Innenseite besetzt ist. Auf die Formkörper wird weiter unten näher eingegangen.

Die Fig. 2 zeigt zudem Stützarme 12, 13, die einerseits an zumindest einer Stelle am Gehäuse 8 befestigt sind, andererseits an der Welle 4, die entlang der strichpunktierten Linie verläuft, welche zudem auch die Zylindersymmetrieachse der Anordnung darstellt.

Die Stützarme 12, 13 sind im komprimierten Zustand in Längsrichtung der Welle 4 angeklappt und befinden sich in einem spitzen Winkel zu dieser.

Wird das Pumpenlaufrad 9 in Betrieb genommen, so dass sich die Pumpe zu drehen beginnt, so entsteht eine Strömung dadurch, dass Flüssigkeit aus der Umgebung des Gehäuses 8 in die Öffnung 14 durch einen Ansaugkäfig 15 angesaugt und zum Katheter 1 hin beschleunigt wird. In dem Pumpengehäuse 8 entsteht damit gegenüber der Umgebung ein Überdruck, der das Pumpengehäuse 8 radial expandiert. In demselben Maß, wie das Pumpengehäuse 8 sich radial ausdehnt, können die Förderschaufeln der Pumpe sich aufstellen und hierdurch kann die Leistung der Pumpe weiter gesteigert werden.

Im unteren Teil der Fig. 2 ist das Gehäuse 8 im voll expandierten Zustand dargestellt, wobei auch das Pumpenlaufrad 9 voll aufgeklappt ist. Die Pumpe bringt in diesem Zustand ihre volle Leistung und die Stützarme 12, 13 sind stumpfwinklig zur Welle 4 abgespreizt.

In diesem Zustand können die Stützarme 12, 13 beispielsweise an ihrem Anlenkpunkt 16 winkelsteif einrasten, so dass sie den expandierten Zustand des Gehäuses 8 stützen.

In dem Zuge der Expansion des Pumpengehäuses 8 findet durch das Spreizen der Stützarme 12, 13 auch eine axiale Bewegung/Kompression des Gehäuses 8 um den Betrag ΔX statt, wie dies in der Fig. 2 angedeutet ist.

In der Fig. 3 ist ein ähnliches Pumpengehäuse 8 wie in der Fig. 2 gezeigt, wobei sich die Stützarme 17, 18 von den in der Fig. 2 gezeigten dadurch unterscheiden, dass jeder Stützarm für sich ein oder mehrere Gelenke 19 aufweist.

Diese Gelenke 19 bewirken eine flexiblere Anpassung der Stützarme 17, 18 an den Expansionsgrad des Gehäuses 8 und führen dazu, dass die axiale Kompression/Verschiebung des Gehäuses 8 während des Aufspannens reduziert oder eliminiert ist.

Auch die gelenkigen Stützarme 17, 18 können in einer bestimmten Position einrasten, so dass auch in dieser Konstellation eine zusätzliche Stützung des expandierten Gehäuses möglich ist. Die eingerastete Stellung kann beim Kollabieren des Gehäuses 8 durch Aufbringen einer bestimmten Schwellenkraft überwunden werden.

Zudem weist die Konstellation der Fig. 3 die Besonderheit auf, dass die Stützarme 17, 18 sich von dem Gehäuse 8 aus nach außen zum Gehäuseäußeren wölben, so dass die Stützarme gleichzeitig einen komfortablen Ansaugkäfig 15 bilden können.

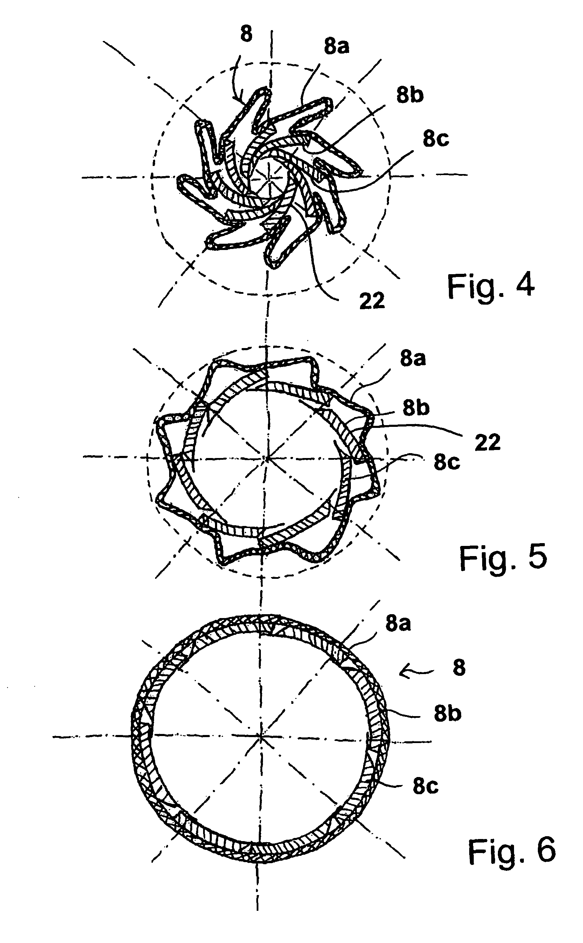

Die Fig. 4 zeigt in einer Querschnittsansicht das Gehäuse 8 in komprimiertem Zustand, wobei in dieser Darstellung deutlich wird, dass das Gehäuse 8 eine Membran 8a und Formkörper 8b, 8c aufweist, die im komprimierten Zustand des Gehäuses zusammengefaltet vorliegen. Die einzelnen Formkörper 8b, 8c sind jeweils nur in einem Teilbereich ihrer Stützfläche 22 durch Klebung mit der Membran 8a verbunden. Der Pumpenrotor ist in der Darstellung der Fign. 4, 5 und 6 der Übersichtlichkeit halber nicht abgebildet.

Die Fig. 5 zeigt das Gehäuse in einem teilweise expandierten Zustand, wobei sich die Formkörper 8b, 8c mit ihren Stützflächen der Membran 8a annähern.

In Fig. 6 ist das Gehäuse 8 in vollständig expandiertem Zustand gezeigt, wobei die Formkörper 8b, 8c an der Membran 8a vollständig anliegen und somit auch eine Strömung innerhalb des Gehäuses nicht behindern. In diesem Zustand berühren die Formkörper 8b, 8c einander und überlappen, so dass sie ineinandergreifen und gegenseitig ihr Position stützen. Sie bilden damit eine schuppenartige Versteifung der Membran und tragen wesentlich zur Stabilität des Gehäuses bei.

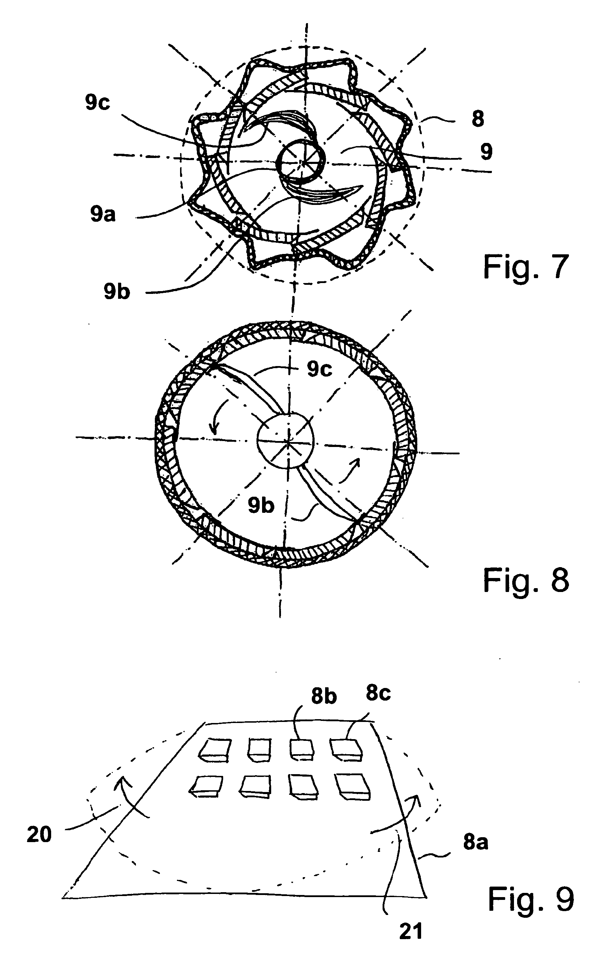

In der Fig. 7 ist im Querschnitt das Pumpengehäuse 8 mit einem Pumpenrotor 9 dargestellt, wobei der Pumpenrotor 9 eine Nabe 9a und Förderschaufeln 9b, 9c aufweist. Die Förderschaufeln 9b, 9c sind wenigstens teilweise an die Nabe 9a angeklappt, was dadurch ermöglicht wird, dass die Förderschaufeln entweder gelenkig an der Nabe befestigt oder in sich elastisch biegbar ausgeführt sind.

In der Darstellung der Fig. 8 ist das Gehäuse 8 vollständig expandiert und die Förderschaufeln 9b, 9c sind maximal aufgerichtet, so dass in dieser Konstellation die maximale Pumpleistung erreicht wird.

In der Fig. 9 schließlich ist eine flache Membran 8a gezeigt, auf der Formkörper 8b, 8c durch Klebung befestigt sind, bevor die Membran 8a, wie durch die Pfeile 20, 21 angedeutet, zu einem Schlauch aufgerollt und zusammengefügt wird.

Die Fig. 10 zeigt vergrößert das Ineinandergreifen der Formkörper 8b, 8c, die asymmetrisch gestaltet sind und schindel- bzw. schuppenartig übereinander liegen.

Durch diese Herstellungsart kann ein erfindungsgemäßes Gehäuse besonders einfach und kostengünstig hergestellt werden. Die Membran kann dabei als biegeschlaffe Folie, elastisch oder nicht elastisch hergestellt sein und die Formkörper 8b, 8c können typischerweise als Kunststoffspritzgussteile realisiert sein. Die schindel- bzw. schuppenartige Positionierung ermöglicht im Bereich der übereinanderliegenden Formteile 8b, 8c einen vorteilhaften Ausgleich der prozessbedingten Abweichungen in Länge und Lage der Formteile. Größere Herstellungstoleranzen können akzeptiert werden und somit Herstellungskosten gesenkt werden. Eine zumindest teilweise automatisierte Fertigung wird ermöglicht.

Das erfindungsgemäße Gehäuse ermöglicht somit bei geringen Kosten und einem geringen konstruktiven Aufwand eine einfache Expandierbarkeit ohne äußeren Kraftaufwand, einfach durch Erzeugung eines Fluiddrucks im Gehäuse. Dies lässt sich insbesondere bei der Verwendung mit einer Pumpe einfach realisieren. Auch beim Komprimieren des Gehäuses ergeben sich praktisch keine Gegenkräfte, so dass das Gehäuse gegebenenfalls mit einem Katheter und gegebenenfalls mit einer Schleuse einfach aus einem Patientenkörper wieder entfernt werden kann.

## Patentansprüche

1. Gerät, enthaltend ein Funktionselement (5) sowie ein Gehäuse (8) für das Funktionselement (5), insbesondere für den Einsatz in körpereigenen Gefäßen im medizinischen Bereich, dessen Gehäusewand eine biegeschlaffe, aufspannbare Membran (8a) aufweist mit mehreren an dieser befestigten Formkörpern (8b, 8c), die die Membran (8a) in deren aufgespanntem Zustand stützen, **dadurch gekennzeichnet, dass** das Gerät eine Fluidpumpe aufweist, die in dem Gehäuse einen Überdruck erzeugt, der zum Aufspannen der Membran führt.

2. Gerät nach Anspruch 1, **dadurch gekennzeichnet, dass** die Formkörper (8b, 8c) zumindest mit der Membran (8a) verbunden sind.

3. Gerät nach Anspruch 1 oder 2, **dadurch gekennzeichnet, dass** wenigstens ein Formkörper (8b, 8c) eine Stützfläche (22) aufweist, die im aufgespannten Zustand an der Membran (8a) anliegt.

4. Gerät nach Anspruch 2 oder 3, **dadurch gekennzeichnet, dass** der wenigstens eine Formkörper (8b, 8c) auf der gesamten Stützfläche (22) mit der Membran (8a) verbunden ist.

5. Gerät nach Anspruch 3, **dadurch gekennzeichnet, dass** der wenigstens eine Formkörper (8b, 8c) nur auf einem Teil der Stützfläche (22), insbesondere nur mit einer Begrenzungskante der Stützfläche mit der Membran (8a) verbunden ist.

6. Gerät nach Anspruch 3, 4 oder 5, **dadurch gekennzeichnet, dass** der Formkörper (8b, 8c) mit der Membran (8a) durch eine Klebeverbindung oder eine andere fügende Verbindung befestigt ist,

7. Gerät nach Anspruch 1, **dadurch gekennzeichnet, dass** die Formkörper (8b, 8c) die Membran (8a) im aufgespannten Zustand auf der Innenseite des Gehäuses im Wesentlichen abdecken.

8. Gerät nach Anspruch 1, **dadurch gekennzeichnet, dass** benachbarte Formkörper (8b, 8c) im aufgespannten Zustand aneinander anliegen.

9. Gerät nach Anspruch 8, **dadurch gekennzeichnet, dass** benachbarte Formkörper (8b, 8c) im aufgespannten Zustand einander teilweise überdecken.

10. Gerät nach Anspruch 8 oder 9, **dadurch gekennzeichnet, dass** benachbarte Formkörper (8b, 8c) im aufgespannten Zustand ineinander greifen.

11. Gerät nach einem der vorhergehenden Ansprüche, **dadurch gekennzeichnet, dass** die Pumpe das Funktionselement (5) bildet.

12. Gerät nach einem der vorhergehenden Ansprüche, **gekennzeichnet durch** eine Ansaugöffnung (15) und einen Katheteranschluss (8d).

13. Gerät nach einem der vorangehenden Ansprüche, **gekennzeichnet durch** einen Pumpenrotor (9) mit wenigstens einer Förderschaufel.

14. Gerät nach einem der vorangehenden Ansprüche, **gekennzeichnet durch** Stützarme (12, 13, 17, 18), die das Gehäuse (8) gegenüber dem Pumpenrotor (9) oder seiner Welle stützen und/oder zentrieren.

15. Gerät nach Anspruch 14, **dadurch gekennzeichnet, dass** die Stützarme biegsam oder an wenigstens einem Knickgelenk knickbar sind.

16. Gerät nach Anspruch 14 oder 15, **dadurch gekennzeichnet, dass** die Stützarme im aufgespannten Zustand der Membran (8a) einrasten.

17. Gerät nach einem der Ansprüche 1 bis 16, **dadurch gekennzeichnet, dass** das Gehäuse so gestaltet ist, dass es nach Ausschaltung der Fluidpumpe kollabiert.

## Claims

1. Device, comprising a functional element (5) and a housing (8) for the functional element (5), in particular for use in the medical field in naturally occurring vessels in the body, the housing wall of the housing (8) has a slack, flexible, tensionable membrane (8a) with a plurality of formed parts (8b, 8c) secured thereon which support the membrane (8a) in the tensioned state thereof,
**characterized in that**
the device has a fluid pump which produces an excess pressure in the housing which leads to tensioning of the membrane.

2. Device according to claim 1, **characterised in that** the formed parts (8b, 8c) are connected at least to the membrane (8a).

3. Device according to claim 1 or 2, **characterised in that** at least one formed part (8b, 8c) has a support surface (22) which abuts against the membrane (8a) in the tensioned state.

4. Device according to claim 2 or 3, **characterised in that** the at least one formed part (8b, 8c) is connected to the membrane (8a) on the entire support surface (22).

5. Device according to claim 3, **characterised in that** the at least one formed part (8b, 8c) is connected to the membrane (8a) only on a part of the support surface (22), in particular only to a delimiting edge of the support surface.

6. Device according to claim 3, 4, or 5, **characterised in that** the formed part (8b, 8c) is secured on the membrane (8a) by an adhesive joint or another joining connection.

7. Device according to claim 1, **characterised in that** the formed parts (8b, 8c) essentially cover the membrane (8a) in the tensioned state on the inside of the housing.

8. Device according to claim 1, **characterised in that** adjacent formed parts (8b, 8c) abut against each other in the tensioned state.

9. Device according to claim 8, **characterised in that** adjacent formed parts (8b, 8c) overlap each other partially in the tensioned state.

10. Device according to claim 8 or 9, **characterised in that** adjacent formed parts (8b, 8c) engage one in the other in the tensioned state.

11. Device according to one of the preceding claims, **characterised in that** the pump forms the functional element (5).

12. Device according to one of the preceding claims, **characterised by** a suction opening (15) and a catheter connection (8d).

13. Device according to one of the preceding claims, **characterised by** a pump rotor (9) having at least one pump blade.

14. Device according to one of the preceding claims, **characterised by** support arms (12, 13, 17, 18) which support and/or centre the housing (8) relative to the pump rotor (9) or its shaft.

15. Device according to claim 14, **characterised in that** the support arms are flexible or bendable at at least one bending joint.

16. Device according to claim 14 or 15, **characterised in that** the support arms engage in the tensioned state of the membrane (8a).

17. Device according to one of the claims 1 to 16, **characterized in that** the housing is configured to collapse after switch off of the fluid pump.

## Revendications

1. Appareil comprenant un élément fonctionnel (5) et un boîtier (8) pour l'élément fonctionnel (5), en particulier destiné à être utilisé à l'intérieur de vaisseaux corporels dans le domaine médical, la paroi du boîtier comportant une membrane (8a) semi-rigide pouvant être tendue, avec plusieurs corps moulés (8b, 8c) fixés sur celle-ci, qui soutiennent la membrane (8a) dans son état tendu, **caractérisé en ce que** l'appareil possède une pompe fluidique qui produit dans le boîtier une surpression qui provoque la tension de la membrane.

2. Appareil selon la revendication 1, **caractérisé en ce que** les corps moulés (8b, 8c) sont reliés au moins à la membrane (8a).

3. Appareil selon la revendication 1 ou 2, **caractérisé en ce qu'**au moins un corps moulé (8b, 8c) possède une surface d'appui (22) qui, dans l'état tendu, repose contre la membrane (8a).

4. Appareil selon la revendication 2 ou 3, **caractérisé en ce que** le au moins un corps de forme (8b, 8c) est relié à la membrane (8a) par l'ensemble de la surface d'appui (22).

5. Appareil selon la revendication 3, **caractérisé en ce que** ledit au moins un corps moulé (8b, 8c) est relié à la membrane (8a) uniquement par une partie de la surface d'appui (22), en particulier uniquement par une arête délimitant la surface d'appui.

6. Appareil selon la revendication 3, 4 ou 5, **caractérisé en ce que** le corps moulé (8b, 8c) est fixé sur la membrane (8a) par un assemblage collé ou un autre assemblage de jonction.

7. Appareil selon la revendication 1, **caractérisé en ce que** les corps moulés (8b, 8c) recouvrent sensiblement la membrane (8a) lorsqu'elle est dans l'état tendu contre la face intérieure du boîtier.

8. Appareil selon la revendication 1, **caractérisé en ce que** des corps moulés (8b, 8c) adjacents, dans l'état tendu, reposent les uns sur les autres.

9. Appareil selon la revendication 8, **caractérisé en ce que** des corps de forme (8b, 8c) adjacents, dans l'état tendu, se chevauchent partiellement les uns les autres.

10. Appareil selon la revendication 8 ou 9, **caractérisé en ce que** des corps de forme (8b, 8c) adjacents, dans l'état tendu, entrent en prise les uns dans les autres.

11. Appareil selon l'une des revendications précédentes, **caractérisé en ce que** la pompe constitue l'élément fonctionnel (5).

12. Appareil selon l'une des revendications précédentes, **caractérisé par** une ouverture d'aspiration (15) et un raccordement de cathéter (8d).

13. Appareil selon l'une des revendications précédentes, **caractérisé par** un rotor de pompe (9) avec au moins une pale de transport.

14. Appareil selon l'une des revendications précédentes, **caractérisé par** des bras d'appui (12, 13, 17, 18) qui soutiennent et/ou centrent le boîtier (8) par rapport au rotor de pompe (9) ou à son arbre.

15. Appareil selon la revendication 14, **caractérisé en ce que** les bras d'appui sont flexibles ou peuvent plier au niveau d'au moins une articulation.

16. Appareil selon la revendication 14 ou 15, **caractérisé en ce que** les bras d'appui s'encliquettent lorsque la membrane (8a) est dans l'état tendu.

17. Appareil selon l'une des revendications 1 à 16, **caractérisé en ce que** le boîtier est configuré pour se rétracter après l'arrêt de la pompe fluidique.
